Europäisches Patentamt

European Patent Office

Office européen des brevets

(19)

(11) Veröffentlichungsnummer : **0 092 688**
**B1**

(12) **EUROPÄISCHE PATENTSCHRIFT**

(45) Veröffentlichungstag der Patentschrift :
22.10.86

(21) Anmeldenummer : 83103155.4

(22) Anmeldetag : 30.03.83

(51) Int. Cl.⁴ : **G 01 N 33/52, G 01 N 33/53**

(54) **Aus mehreren Schichten bestehender Schichtkörper und Verfahren zum Nachweis und/oder Messen der Konzentration einer chemischen Substanz, insbesondere biologischer Herkunft.**

(30) Priorität : 26.04.82 DE 3215484

(43) Veröffentlichungstag der Anmeldung :
02.11.83 Patentblatt 83/44

(45) Bekanntmachung des Hinweises auf die Patenterteilung : 22.10.86 Patentblatt 86/43

(84) Benannte Vertragsstaaten :
AT BE CH FR GB IT LI NL SE

(56) Entgegenhaltungen :
DE-A- 2 638 250
US-A- 3 926 564
US-A- 3 979 184
US-A- 4 181 501
Die Akte enthält technische Angaben, die nach dem Eingang der Anmeldung eingereicht wurden und die nicht in dieser Patentschrift enthalten sind.

(73) Patentinhaber : SAGAX Instrument AB
Box 7003
S-172 07 Sundbyberg (SE)

(72) Erfinder : Nygren, Bo Hakan, Dr.
Bredängen 40
S-427 00 Billdal (SE)
Erfinder : Sandström, Erland Torbjörn
Muraregatan 13B
S-431 36 Mölndal (SE)
Erfinder : Stenberg, Johan Emanuel, Dr.
Hedelundsvägen 5
S-417 43 Göteborg (SE)
Erfinder : Stiblert, Lars Bertil, Dr.
Övre Besvärsgatan 4
S-411 29 Göteborg (SE)

(74) Vertreter : Nöth, Heinz, Dipl.-Phys.
Patentanwalt Müllerstrasse 1
D-8000 München 5 (DE)

**Beschreibung**

Die Erfindung betrifft einen aus mehreren Schichten bestehenden Schichtkörper sowie ein Verfahren zum Nachweis und/oder Messen der Konzentration einer chemischen Substanz, insbesondere biologischer Herkunft in einem Medium, z. B. Wasser, Gel, Blutserum, mit einem nichtmetallischen Träger, der mit einer dielektrischen Schicht versehen ist, auf welcher ein mit der nachzuweisenden Substanz reagierendes Nachweisreaktionsmittel aufgebracht ist, auf welchem sich beim Einbringen in das zu untersuchende Medium eine optisch nachweisbare Schicht der nachzuweisenden Substanz bildet.

Bei biochemischen und immunologischen Untersuchungen besteht ein großes Bedürfnis zur Erfassung und Messung dünner organischer Oberflächenschichten, insbesondere solcher Oberflächenschichten mit Brechungsindizes um 1,50 und Dicken im Bereich von 1-10 nm. Diese Untersuchungen sind insbesondere von Interesse in Verbindung mit Toxin-Rezeptor-, Enzym-Substrat- oder Antigen-Antikörperreaktionen. Es ist bekannt, daß eine Antigen-Antikörperreaktion, d. h. eine Bindung eines Antigens an seinen spezifischen Antikörper, auch dann stattfindet, wenn einer der beiden Reaktionsteilnehmer an eine Oberfläche gebunden ist. Es ist daher möglich, ein Protein eines Mikroorganismus an eine Oberfläche zu binden und die Oberfläche in ein Blutserum einer Person einzutauchen. Wenn diese Person infiziert ist durch einen Mikroorganismus und im Blutserum Antikörper gegen die Proteine des Mikroorganismus enthalten sind, werden diese Antikörper am Protein, das auf der eingetauchten Oberfläche vorhanden ist, gebunden, wenn dieses Protein vom gleichen Typ ist wie die Proteine des Mikroorganismus. Auf diese Weise wächst eine organische Schicht auf der eingetauchten Oberfläche. Gleichfalls kann natürlich auch ein Antikörper gegen ein bestimmtes Protein an die eingetauchte Oberfläche gebunden sein und zur Erfassung des Proteins in der Lösung verwendet werden. Diese Untersuchungsverfahren haben in neuerer Zeit immer mehr an Bedeutung gewonnen, insbesondere weil monoklonale Antikörper gegen einen breiten Bereich von Proteinen durch hybridome Techniken hergestellt werden können. Ein Vorteil der Oberflächenreaktionen im Vergleich zu Reaktionen in flüssigen Phasen ist der, daß nur ein geringerer Verbrauch an Reaktionsmitteln erfolgt.

Die Anwendung von Antikörpern bzw. Antigenen, die an Oberflächen unbeweglich gemacht sind bzw. an Oberflächen gebunden sind bei immunologischen Untersuchungen ist jedoch beeinträchtigt durch das Fehlen einfacher und empfindlicher Meßverfahren für die Dicke der organischen Filme im nm-Bereich. Im Laborbereich wird hierzu die Ellipsometrie verwendet. Diese ist jedoch relativ aufwendig und kann nur von qualifiziertem Personal durchgeführt werden. Einfachere Untersuchungstechniken, welche beispielsweise auf der Änderung der Benetzbarkeit der Lichtstreuung von Metallpartikeln und des Haftvermögens von Kolloidpartikeln beruhen, haben sich in der Praxis nicht durchsetzen können. Bei diesen vereinfachten Verfahren wird die unterschiedliche Ausbildung zwischen einem begrenzten Flächenbereich und die ihn umgebende Fläche ausgenützt. Dabei wird der begrenzte Flächenbereich mit einem Antigen oder Antikörper beschichtet, während die umgebende Fläche mit einer anderen organischen Substanz der gleichen Dicke beschichtet ist. Wenn Proteine im begrenzten Flächenbereich gebunden wurden und nicht an der diesen umgebenden Fläche, ergibt sich im begrenzten Flächenbereich eine größere Schichtdicke als in der Umgebung. Hieraus resultieren unterschiedliche physikalische Eigenschaften. Antigen-Antikörperreaktionen und dielektrische Schichten auf reflektierenden Trägern wurden von Blodgett und Langmuir bereits vor längerer Zeit untersucht (Physical Review, Bd. 51, Juni 1937, S. 964 bis 978).

Diese Untersuchungen zeigen, daß dünne organische Filme auf polierten Chromoberflächen beobachtet werden können, wenn sie nicht direkt auf die blanke Oberfläche, sondern auf einen auf der Chromoberfläche gebildeten Bariumstearatfilm aufgebracht werden. Starke Interferenzen können wegen nicht optimaler Brechungsindizes bei senkrechtem Einfallswinkel nicht erzielt werden. Die Interferenz ist stärker bei großen Einfallswinkeln, jedoch ist dann das Auftreten der beiden Polarisationszustände unterschiedlich. Eine starke Unterdrückung einer einzelnen Wellenlänge kann daher nur durch gleichzeitige Verwendung eines Polarisators erzielt werden. Beim Betrachten der Probe durch einen Polarisator und durch Messung des Einfallswinkels bei minimaler Intensität läßt sich die Dicke monomolekularer Filme nach Blodgett und Langmuir relativ genau bestimmen und damit ein Nachweis erbringen für eine Bindungsreaktion zwischen Antigene und Antikörper auf Oberflächen. Man hat hier unter Anwendung eines rudimentären Ellipsometrieverfahrens immunologische Reaktionen untersucht.

Ferner ist der Gebrauch starker Interferenzen von Tantaloxid auf Tantal durch Vroman bekannt geworden (Thromb. Diath. Haemorrhag., Bd. 10, S. 455 bis 493 (1964), insbesondere Seite 463.

Eloxierte Tantalplatten mit starkbrauner Einfärbung wechseln nach Violett, wenn sie mit einer monomolekularen Proteinschicht überzogen werden. Das Oxid bildet einen reflexionsmindernden Überzug auf dem Metall, und die Platte hat ein braunes Aussehen, wenn die Reflexion von blauem und grünem Licht stärker unterdrückt wird als die Reflexion größerer Wellenlängen. Bei der Bildung einer dünne Proteinschicht auf der Oberfläche wächst die Schichtdicke der durchlässigen Schicht, und das Reflexionsminimum wandert zu größeren Wellenlängen. Das reflektierte Licht enthält daher mehr blaue und violette Anteile, wenn eine Bioschicht, z. B. Proteinschicht, vorhanden ist.

Gleiche Eigenschaften wie bei Vroman wurden von Giaever und Laffin (Proc. Nat. Acad. Sci. USA, Vol. 71, No. 11, S. 4533-4535, November 1974) mit einem eingangs genannten Schichtkörper erzielt, von welchem im Oberbegriff des Anspruchs 1 bzw. 12 ausgegangen wird. Bei diesem bekannten Schichtkörper wird eine Gold-Indiumlegierung auf einen als Glasschieber ausgebildeten Träger aufgebracht und allmählich zu einer dielektrischen Schicht oxidiert, so daß eine bräunliche Einfärbung entstand. Auf die dielektrische Oxidschicht wird dann ein Antigen als Nachweisreaktionsmittel aufgebracht.

Bei den drei letztgenannten bekannten Verfahren wurde der Effekt ausgenützt, daß dünne Bioschichten, insbesondere Proteinschichten, welche auf dielektrische Schichten mit bestimmter Dicke aufgebracht werden, die Interferenzen der ursprünglichen dielektrischen Schicht ändern. Bei den bekannten Verfahren kommen Metalle zum Einsatz, welche dann, wenn sie als Träger verwendet werden, darauf befindliche Filme mit hohem Brechungsindex benötigen, wenn gute Interferenzeigenschaften erzielt werden sollen, wobei jedoch eine geringe optische Nachweisempfindlichkeit in Kauf genommen wird. Ferner besteht die Gefahr, daß durch die Berührung mit dem Metall bzw. Metalloxiden die Reaktionsmittel beeinflußt werden. Dies ist insbesondere dann unerwünscht, wenn nur geringe Konzentrationen der nachzuweisenden Substanz in den Lösungen vorhanden und lange Inkubationszeiten notwendig sind. Ferner besitzen Metallträger bzw. darauf befindliche Metalloxidschichten unbestimmte Oberflächeneigenschaften, da die Oberflächenenergie und die Oberflächendichte der Bindungsstellen sich ändert und deshalb die Bindung bzw. Adsorption organischer Moleküle nicht genau genug gesteuert werden kann.

Auch bei dem aus der DE-OS 26 38 250 bzw. aus « The Journal of Immunology », Vol. 110, Nr. 5, Mai 1973, S. 1424 bis 1426 bekannten Nachweissystem von Antikörpern und Antigenen handelt es sich um ein metallisches System, bei dem auf ein nichtmetallisches Substrat, beispielsweise aus Glas, Glimmer, Kunststoff, Siliciumdioxid, Quarz und ähnlichem, Metallkügelchen aus Indium, Gold, Silber, Zinn, Blei oder einem Metall mit ähnlichen Eigenschaften aufgedampft werden. Hierauf wird eine monomolekulare Schicht eines Antigens aufgebracht, wobei gegebenenfalls vor dem Aufbringen des Antigens die Metallkügelchen noch oxidiert werden.

Aus den US-Patenten 3 926 564 und 3 979 184 sind zur Sichtbarmachung dünner Oberflächenschichten ebenfalls metallische Systeme bekannt, wobei in der US-PS 3 979 184 eine dielektrische Schicht zwischen einem metallischen Substrat und einer halbdurchlässigen Metallschicht angeordnet ist und in der US-PS 3 926 564 ein Oxid auf einer Edelmetall-Legierung mit einem oxidierbaren Bestandteil gebildet ist. Hierbei handelt es sich ebenfalls um metallische Systeme, bei denen, wie schon erwähnt, eine geringe optische Nachweisempfindlichkeit in Kauf genommen wird.

Ferner ist es bekannt (Laurell, C. B. « Quantitative estimation of protein by elektrophoreses in agarose gel containing antibodies », Anal. Biochem. 15 : 45, 1966), die nachzuweisende Substanz bzw. deren Menge durch Elektrophorese in einem Gel sich ausbreiten zu lassen, welches eine homogen verteilte Konzentration eines Reaktionsmittels, das mit der nachzuweisenden Substanz reagiert, enthält. Die beiden Reaktionsmittel reagieren durch Ausfällung im Gel und die Reaktionszone wird sichtbar gemacht, wobei die. Reaktionszone begrenzt ist auf den Bereich, in welchem das Reaktionsmittel für die nachzuweisende Substanz von Anfang an enthalten ist. Man erzielt hierdurch einen raschen und genauen Nachweis. Diese Nachweismethode ist jedoch dadurch eingeschränkt, daß das Reaktionsmittel, welches für den Nachweis verwendet wird, im elektrischen Feld nicht wandern darf, wodurch hinsichtlich der Elektrophoresebedingungen, wie beispielsweise pH, der Gelqualität un der Polarität Beschränkungen vorhanden sind. Ferner ist das Verfahren beschränkt auf solche Reaktionen, welche zu einer Ausfällung führen.

Bei einem weiteren bekannten Verfahren (Elwing, H. and Nygren, H. « Diffusion in gelenzyme linked immunosorbent assay (DIG-ELISA) : A simple method for quantification of classspecific antibodies », J. Immun. Methods, 31 : 101, 1979) breiten sich die nachzuweisenden Substanzen durch Diffusion in einem Gel aus, das über einer Oberfläche angeordnet ist, die mit einer dünnen Schicht eines Reaktionsmittels zum Nachweis der Substanz versehen ist. Nach einer bestimmten Zeit wird das Gel entfernt und die Reaktionszone auf der Oberfläche sichtbar gemacht. Die Größe der Reaktionszone wird gemessen und ist ein Maß für die Menge der nachzuweisenden Substanz, welche auf der Oberfläche diffundiert ist. Die Sichtbarmachung des Reaktionsprodukts an der Oberfläche kann durch sekundäre Reaktionen, wie beispielsweise durch Inkubation mit einem Isotop oder enzymmarkierten Antikörpern durchgeführt werden, welche gegen das nach der Diffusion gebundene Reaktionsprodukt gerichtet werden. Auch ist es möglich, den Nachweis durch ein Wasserkondensationsverfahren (Adams, A. L., Klings, M., Fischer, G. C. and Vroman, L., « Three simple ways to detect antibody antigen complex on flat surfaces », J. Immun. Methods, 3 : 227, 1973) zu führen. Ferner läßt sich die Sichtbarmachung durch direkte optische Analyse des primären Reaktionsprodukts, durch Ellipsometrie (Elwing, H. and Stenberg, M. « Biospecific bimolecular binding-reactions. A new ellipsometric method for their detection, quantification and characterization », J. Immun. Methods, 44 : 343, 1981) oder durch Lichtinterferenzen an dünnen Schichten (Adams, A. L., Klings, M., Fischer, G. C. and Vroman, L., « Three simple ways to detect antibody antigen complex on flat surfaces », J. Immun. Methods, 3 : 227, 1973) durchführen. Bei der Sichtbarmachung wird durch Verstärkungsreaktionen, wie beispielsweise durch Einsatz von enzymmarkierten Antikörpern, der Vorteil einer hohen Empfindlichkeit erzielt, während die optischen Nachweismethoden einen direkten Nachweis des primären Reaktionsprodukts liefern. Außerdem wird bei den optischen Nachweismethoden kein anderes Reaktionsmittel als das zum Nachweis benötigte Reaktionsmittel verwendet.

Bei dem Diffusionsverfahren, bei dem eine Oberfläche mit einem Reaktionsmittel zum Nachweis einer Substanz aufgebracht ist, ergibt sich gegenüber der passiven oder aktiven Transportreaktion eines im Gel verteilten Reaktionsmittels der Vorteil, daß auch Reaktionen, die nicht zu einer Ausfällung führen, untersucht bzw. nachgewiesen werden können. Zum Unbeweglichmachen des Reaktionsteilnehmers auf der Oberfläche gibt es eine Reihe von Möglichkeiten, die Moleküle des Reaktionsteilnehmers, der für den Nachweis der anderen Substanz verwendet werden soll, zu binden. Bei den Diffusionsverfahren benötigt man jedoch eine relativ lange Diffusionszeit. Bei dem aus FEPS Letters, Band 135, Nr. 1, November 1961, Seiten 73 bis 76 bekannten Verfahren ist es jedoch möglich, in relativ kurzer Zeit mit wenig Aufwand die nachzuweisende Substanz mit dem auf der Trägeroberfläche vorhandenen Reaktionsteilnehmer durch Bindung zur Reaktion zu bringen. Ferner sind die Elektrophorese-Bedingungen keinerlei Beschränkungen unterworfen. Schwierigkeiten ergeben sich jedoch noch hinsichtlich einer Vereinfachung der Sichtbarmachung der nachzuweisenden Substanz, welche auf dem an der Trägeroberfläche vorhandenen Bindemittel gebunden ist. Ferner besteht die Gefahr bei metallischen Trägern, daß unerwünschte elektrochemische Reaktionen, beispielsweise die Bildung von Oxiden auf der Trägeroberfläche, stattfinden.

Biomedizinische Verfahren zum Nachweis organischer Substanzen in Lösungen sind außerdem bekannt aus der DE-OS 25 12 730 und der US-PS 41 81 501. Aus der DE-OS 25 12 730 ist die radiale Diffusion der nachzuweisenden Substanz in einem Gel und der Transport der nachzuweisenden Substanz mittels Elektrophorese in einem Gel bekannt, jedoch erfolgt der Nachweis der Substanzen mit Hilfe metallischer Plättchen, bei denen, wie im vorstehenden schon erwähnt, eine relativ geringe Nachweisempfindlichkeit in Kauf genommen wird.

Aufgabe der Erfindung ist es demgegenüber, einen aus mehreren Schichten bestehenden Schichtkörper, insbesondere in Plättchenform, sowie ein Verfahren der eingangs genannten Art zu schaffen, bei denen eine hohe optische Nachweisempfindlichkeit ohne Beeinträchtigung der chemischen Nachweisreaktion durch das für den Schichtkörper verwendete Material erzielt wird.

Diese Aufgabe wird vorrichtungsmäßig durch die im Anspruchs 1 und verfahrensmäßig durch die im Anspruchs 12 angegebenen Merkmale gelöst.

Die Erfindung macht sich die Erkenntnis zunutze, daß das von einer reflektionsmindernden Oberfläche, welche mit weißem bzw. nichtmonochromatischem Licht bestrahlt wird, reflektierte restliche Licht ein Instrument mit hoher Nachweisempfindlichkeit sein kann für Änderungen der Eigenschaften des reflektionsmindernden Überzugs, wenn, wie bei der Erfindung, alle Oberflächenschichten, d. h. sowohl die dielektrischen Schichten als auch die darauf aufgebrachten bzw. darauf entstehenden Schichten biologischen Ursprungs den reflektionsmindernden Überzug bilden. Es lassen sich dabei geringste Änderungen der Schichtdicke, insbesondere Schichtdickenänderungen im Molekularbereich, durch Farbänderungen im reflektierten Licht feststellen.

In vorteilhafter Weiterbildung der Erfindung kann daher der Schichtkörper so ausgebildet sein, daß an der Oberfläche der Schichtanordnung nur stellenweise das Nachweisreaktionsmittel vorhanden ist. Bevorzugt kann diese Anordnung so sein, daß das Nachweisreaktionsmittel inselförmig in der Schichtanordnung vorhanden ist und von einer nichtaktiven organischen Substanzschicht gleicher Dicke und optischen Eigenschaften umgeben ist. Dabei sind sowohl die Nachweisreaktionsmittelschicht als auch die nichtaktive organische Substanzschicht monomolekular auf die dielektrisch wirkende Schicht aufgebracht, wobei als dielektrische Schicht eine $SiO_2$-Schicht bevorzugt geeignet ist.

Hierbei kann ausgenützt werden, daß eine $SiO_2$-Oberfläche chemisch so ausgebildet sein kann, daß gute Bindungseigenschaften zu verschiedenen organischen Molekülen hergestellt werden können. Wenn der Siliciumkörper bzw. -träger mit der $SiO_2$-Oberfläche in eine ein Protein enthaltende Lösung eingebracht wird, wird die Oberfläche der Dioxidschicht mit einer monomolekularen Schicht des Proteins überzogen. Der beschichtete Siliciumkörper bzw. -träger kann dann getrocknet und auch über einen langen Zeitraum hin ohne Verlust des Proteinüberzugs gelagert werden. Wenn der beschichtete Körper in eine andere Lösung eingebracht wird, kann der Proteinüberzug mit Substanzen in dieser Lösung reagieren, z. B. mit Antikörpern, die dann am Protein gebunden werden. Das Siliciumdioxid ist inert und gibt keine Atome oder Atomgruppen ab, welche an diesen Reaktionen teilnehmen oder welche die Proteine beeinträchtigen. Demzufolge besitzt die Außenfläche aus Siliciumdioxid beim Schichtkörper inerte, jedoch chemisch vielseitig im Sinne der Erfindung anwendbare Eigenschaften.

Eine reproduzierbare $SiO_2$-Oberfläche kann durch Reinigung in einer leicht oxidierenden Lösung erzielt werden. Mit Hilfe bekannter Verfahren können dabei ein breites Spektrum reproduzierbarer Oberflächeneigenschaften erreicht werden. Funktionelle Gruppen, wie beispielsweise Aminogruppen, können dabei kovalent an die Oberfläche mit Hilfe im Handel erhältlicher Kopplungsmittel, z. B. Organo-Siliciumverbindungen, gebunden werden. Größere organische Moleküle, wie beispielsweise Proteine, können auf diese Weise an den funktionellen Gruppen gebunden werden. Hierdurch läßt sich die Oberfläche mit einer monomolekularen kovalent gebundenen organischen Schicht, insbesondere Proteinschicht, überziehen. In vielen Fällen reicht es aus, die $SiO_2$-Oberfläche mit Dichlordimethylsilan zu behandeln. Man gewinnt hierdurch eine stark hydrophobische Oberfläche, auf der die größeren organischen Moleküle, insbesondere Proteine, welche die Nachweisreaktionsmittelschicht bilden, stark adsorbiert werden können.

Der Träger kann aus Silicium bestehen und an der Oberfläche, über welcher die $SiO_2$-Schicht sich befindet, mit der reflektionsmindernden Beschichtung versehen sein. Gegenüber Metallträgern, welche zwar ein hohes Reflektionsvermögen und damit ein gefälliges Aussehen und die Möglichkeit der Verwendung der Metalloxide als reflektionsmindernde Beschichtung besitzen, hat bei der Erfindung der Träger die Vorteile, daß er mit der Lösung nicht reagiert und somit keine Atome bzw. Atomgruppen des Trägers in die Lösung gelangen. Die biochemischen Eigenschaften werden nicht beeinflußt, was zu fehlerhaften Ergebnissen führen könnte. Während Metalle wellenlängenabhängige Brechungsindizes aufweisen und somit das hohe Reflektionsvermögen starke Einschränkungen bezüglich der Wahl des reflektionsmindernden Überzugs bedingt, besitzt das bei der Erfindung zur Anwendung kommende dielektrische Material einen niedrigeren Brechungsindex, der weniger stark von der Wellenlänge abhängt. Als geeignete Träger erweisen sich auch solche aus Glas oder Kunststoff. Diese sind im Handel ohne weiteres erhältlich und mechanisch stabil. Sie sind chemisch inert und können durch chemische Mittel gereinigt werden.

Das nichtmetallische Trägermaterial besitzt einen im wesentlichen dielektrischen Brechungsindex, bei dem der Imaginärteil gegenüber dem Realteil vernachlässigbar klein ist, während bei den Brechungsindizes der Metalle die Realteile und Imaginärteile größenordnungsmäßig gleiche Größe haben.

Langmuir und Blodgett (Physical Review Band 51, Juni 1937, Seiten 964 bis 978) untersuchten zwar ebenfalls Bariumstearat auf Glas, jedoch ergab sich, daß die Interferenzen noch bei schrägen Einfallswinkeln auch unter Zuhilfenahme eines Polarisators schwächer waren als an Chrom. Das Bariumstearat auf Glas ist zwar optisch verwandt zu $SiO_2$ auf Glas, jedoch ist die Interferenz in der praktischen Anwendung zu gering ohne die bei der Erfindung zur Anwendung kommenden dielektrischen Zwischenschichten. Darüber hinaus besitzt das Bariumstearat auf dem Glasträger nicht die erwünschten Oberflächeneigenschaften wie die $SiO_2$-Schicht.

Berechnungen haben ergeben, daß ein reflektionsmindernder Überzug aus einer einzelnen Schicht, d. h. ein Überzug mit hoher Unterdrückung der Reflektion, an einer bestimmten Wellenlänge in der Nähe des Empfindlichkeitsmaximums des Auges (570 nm) den Oberflächenschichten eine hohe Nachweisempfindlichkeit gibt. Für eine Siliciumdioxidschicht, welche eine gute Unterdrückung des reflektierten Lichts gewährleistet, muß der Träger einen Brechungsindex in der Größenordnung von 2,13 aufweisen. Gläser, welche im Sinne der Erfindung gute Trägereigenschaften besitzen, haben Brechungsindizes im Bereich von 1,45-1,90 und die meisten Kunststoffe haben etwa 1,50. Diese Schwierigkeit kann dadurch überwunden werden, daß zwischen dem dielektrischen $SiO_2$ und dem Träger eine Zwischenschicht angeordnet ist, deren Brechungsindex sich unterscheidet gegenüber denen des Siliciumoxids und des Trägers und für eine bestimmte Wellenlänge (insbesondere 570 nm) nur eine geringe Reflektion aufweist. Die Dicke der beiden Schichten (der Zwischenschicht und der dielektrischen $SiO_2$-Schicht) hängt ab von den Brechungsindizes der Zwischenschicht und des Substrats.

Da die Vorderseite der Platte bzw. die Oberfläche des Trägers, über welcher sich die $SiO_2$-Schicht befindet, reflektionsmindernd beschichtet ist, muß die von der Rückseite des Trägers bewirkte Reflektion unterdrückt werden. Dies läßt sich dadurch erzielen, daß ein allmählich lichtabsorbierender Träger verwendet wird, z. B. dunkel eingefärbtes Glas. Die Farbe kann dabei gleichförmig im Träger verteilt sein oder innerhalb einer eingefärbten Schicht mit dem gleichen Brechungsindex wie der übrige Träger enthalten sein. Insbesondere eignen sich Halbleiter, welche optisch als adsorbierende Dielektrika mit hohen Brechungsindizes in Erscheinung treten, wie z. B. Silicium.

In vorteilhafter Weise lassen sich somit immunologische Untersuchungen ohne die Verwendung von zusätzlichen Instrumenten durchführen, wobei billige Stoffe für die Nachweiskörper verwendet werden können. Man erhält einen für das bloße Auge visuell feststellbaren Nachweis für dünne organische Schichten. Die insbesondere plättchenförmigen Nachweiskörper können mit herkömmlichen Techniken ohne weiteres bei hoher Reproduzierbarkeit hergestellt werden. Die Nachweiskörper besitzen eine hohe Nachweisempfindlichkeit, eine einfache Anwendbarkeit und können mit geringem Aufwand in industriellem Maßstab, d. h. in Massenfertigung, hergestellt werden.

Bei dem visuellen Nachweis ist es lediglich notwendig, daß der dem Serum entnommene Nachweiskörper mit weißem Licht an der Seite bestrahlt wird, auf welcher die Schichtanordnung vorhanden ist und das weiße Licht hier zur Reflektion gebracht wird. Für den Fall, daß auf der Nachweisreaktionsmittelschicht sich z. B. eine Antigen- bzw. Antikörperschicht abgelagert hat, gewinnt man gute Kontraste, die mit bloßem Auge sichtbar sind. Diese lassen sich mit hoher Gleichförmigkeit und Reproduzierbarkeit erzielen. Die chemisch inerte Oberfläche des Nachweiskörpers beeinflußt die bei chemischen Reaktionen, insbesondere Antigen-Antikörperreaktionen nicht. Die chemischen Eigenschaften der entsprechend beschichteten Oberfläche des Trägers erleichtern das Binden verschiedener Proteine. Der Nachweiskörper ist physikalisch stabil und hat eine hohe Lebensdauer. Es lassen sich auf diese Weise die immunologischen Untersuchungen mit äußerst geringem Aufwand durchführen, da keine Spezialgeräte notwendig sind.

In vorteilhafter Weise kommt als Dielektrikum Siliciumdioxid mit den für die Erfindung besonders gut geeigneten Oberflächeneigenschaften als Dielektrikum zum Einsatz, wobei als Träger ein inertes dielektrisches Material Verwendung finden kann. Zur Erzielung der erwünschten optischen Eigenschaften kann zwischen dem Dielektrikum aus Siliciumdioxid und dem Träger eine weitere dielektrische Zwischenschicht, insbesondere aus SiO, angeordnet sein. Der Träger besitzt ein ausreichendes Absorptionsvermögen und hat bei Durchstrahlung ein dunkles Aussehen.

Durch das Verfahren wird ein vereinfachter optischer Nachweis der nachzuweisenden Substanz und durch die elektrischen Schichten eine elektrische Isolierung gegenüber dem halbleitenden Träger erzielt.

Da die Reaktion zwischen der nachzuweisenden Substanz und dem Nachweisreaktionsmittel innerhalb eines bestimmten Flächenbereichs auf der Schichtanordnung stattfindet, kann in Abhängigkeit von den geometrischen Abmessungen der Reaktionszone eine Aussage hinsichtlich der Konzentration oder Menge der nachzuweisenden Substanz in der Probenlösung gemacht werden. Auch ist es möglich, eine Konzentrations- bzw. Mengenbestimmung durchzuführen in Abhängigkeit von der Dicke des Gelkörpers, wenn dieser beispielsweise mit keilförmigem Querschnitt auf der Schichtanordnung aufgebracht ist und die Probenlösung mit der darin befindlichen nachzuweisenden Substanz durch Diffusion durch den Gelkörper in Richtung auf die oben liegende Nachweisreaktionsmittelschicht transportiert wird.

Die Erfindung läßt sich in vorteilhafter Weise anwenden bei der Bestimmung solcher biochemischer Substanzen, welche einen der Reaktionsteilnehmer in einer biospezifischen, biomolekularen Reaktion bilden, beispielsweise bei Antigen-Antikörperreaktionen, Enzym-Trägerreaktionen oder Toxin-Rezeptorreaktionen.

Bei der Erfindung läßt sich eine Nachweisempfindlichkeit erzielen, die nahe dem theoretischen Optimum liegt. Diese hohe Nachweisempfindlichkeit ergibt sich durch die Kombination der starken Interferenzwirkung und der starken optischen Kopplung bzw. der Einbeziehung der Bioschicht in die Interferenz. Letzteres läßt sich nur erzielen bei relativ geringer Reflexion. Metalle sind diesbezüglich im Nachteil gegenüber dielektrischen Substraten, welche bei der Erfindung Anwendung finden.

Die chemischen Eigenschaften der Siliciumdioxidoberfläche sind vielseitig, können jedoch genau festgelegt werden. Ein breiter Bereich organischer Moleküle kann daher als Nachweisreaktionsmittel an der $SiO_2$-Oberfläche durch Adsorption oder kovalente Bindung unter Zuhilfenahme von Organo-Siliciumverbindungen als Kopplungsmittel gebunden werden. Beispielsweise kann die $SiO_2$-Oberfläche stark hydrophobisch durch Behandlung mit Dichlordimethylsilan gemacht werden. Hierdurch gewinnt man eine Oberfläche mit starker Adsorptionswirkung auf viele Proteinmoleküle.

Die bei der Erfindung zur Anwendung kommenden Nachweiskörper sind chemisch inert. Sie können gereinigt und mit Hilfe von oxidierenden Säuren regeneriert werden. Die Gefahr der Abgabe von Metallionen während einer verlängerten Inkubationszeit, welche zur Verunreinigung der organischen Substanzen führen, besteht nicht.

Die Herstellung der Nachweiskörper in Plättchenform kann in industriellem Maßstab erfolgen. Die dielektrischen Schichten können bevorzugt aufgedampft oder aufgesprüht werden. Diese Verfahren sind einfacher und genauer durchführbar als die Eloxierung oder Verdampfung körniger Metallschichten oder die thermische Oxidation metallischer Legierungen. Die optischen Anforderungen der erfindungsgemäßen Nachweiskörper sind äußerst gering und die Abmessungen der Nachweiskörper lassen sich in Abhängigkeit von den Anforderungen jeweils festlegen.

Anhand von Ausführungsbeispielen wird die Erfindung noch erläutert.

Beispiel 1

Auf ein Siliciumplättchen wird Siliciummonoxid aufgedampft. Die Oberflächenschicht des Siliciummonoxids wird durch die Umgebungsluft zu Siliciumdioxid oxidiert. Die Schichtdicke von $SiO_2$ beträgt 2 bis 3 nm und von SiO 60 bis 70 nm.

Auf diese Weise wurde ein Ausgangskörper für den endgültigen Nachweiskörper geschaffen.

Beispiel 2

Siliciummonoxid wird auf eine dunkel eingefärbte Glasplatte in einer Dicke von 60 bis 70 nm durch Aufdampfen aufgebracht. Daraufhin wird Sauerstoffgas in die Verdampfungskammer eingeleitet, und es wird weiterhin Siliciummonoxid verdampft. In Anwesenheit des Sauerstoffgases schlägt sich Siliciumdioxid nieder und der Verdampfungsvorgang wird beendet, sobald die $SiO_2$-Schicht eine Dicke von 90 bis 100 nm erreicht hat.

Die $SiO_2$-Schichten auf beiden Nachweiskörpern des Beispiels 1 und des Beispiels 2 sind etwas dünner bemessen als für die optimale Unterdrückung des reflektierten Lichtes notwendig ist. Begrenzte inselförmige Oberflächenbereiche der $SiO_2$-Schicht wurden mit monomolekularen Schichten aus Proteinen bzw. Antikörpern beschichtet. Die übrigen Oberflächenteile der jeweiligen $SiO_2$-Schicht wurden

mit hiervon unterschiedlichen Proteinen beschichtet, so daß ein optisch gleichförmiger Überzug auf der gesamten Oberfläche des Dielektrikums vorhanden war. Die SiO$_2$-Schichten wurden jeweils etwas dünner ausgestaltet als es für die optimale Unterdrückung des reflektierten Lichts notwendig war und die darauf aufgebrachten gleichförmigen organischen Schichten waren so bemessen, daß die Reflektion nahe dem Reflektionsminimum liegt.

Die Platten wurden daraufhin in eine Lösung eingebracht, welche organische Moleküle aufwiesen, die mit den organischen Schichten in den inselförmig begrenzten Oberflächenbereichen reagierten. Die Bindung von Molekülen an diesen aktiven inselförmigen Oberflächenbereichen bewirkte eine Erhöhung der Dicke in Abhängigkeit von den verwendeten Substanzen von 1-5 nm. Die Schichtplättchen wurden aus der Lösung entnommen und getrocknet. Es ergab sich dann, daß die inselförmigen Bereiche klar sichtbar mit bloßem Auge waren und etwas dunkler und mehr purpurn waren als die umgebende Oberfläche, wenn weißes Licht zur Reflektion gebracht wurde. Es konnten Konzentrationen in bis zu 1 ng/ml-Lösungen auf diese Weise noch leicht festgestellt werden.

Die beiliegenden Figuren zeigen schematisch Ausführungsbeispiele und dienen der weiteren Erläuterung.

Es zeigt :

Figur 1 ein erstes Ausführungsbeispiel für einen Schichtkörper ;

Figur 2 ein zweites Ausführungsbeispiel für einen Schichtkörper ;

Figur 3 bis 5 weitere Ausführungsbeispiele für den Transport der nachzuweisenden Substanz durch einen Gelkörper mit Hilfe von Elektrophorese oder Diffusion.

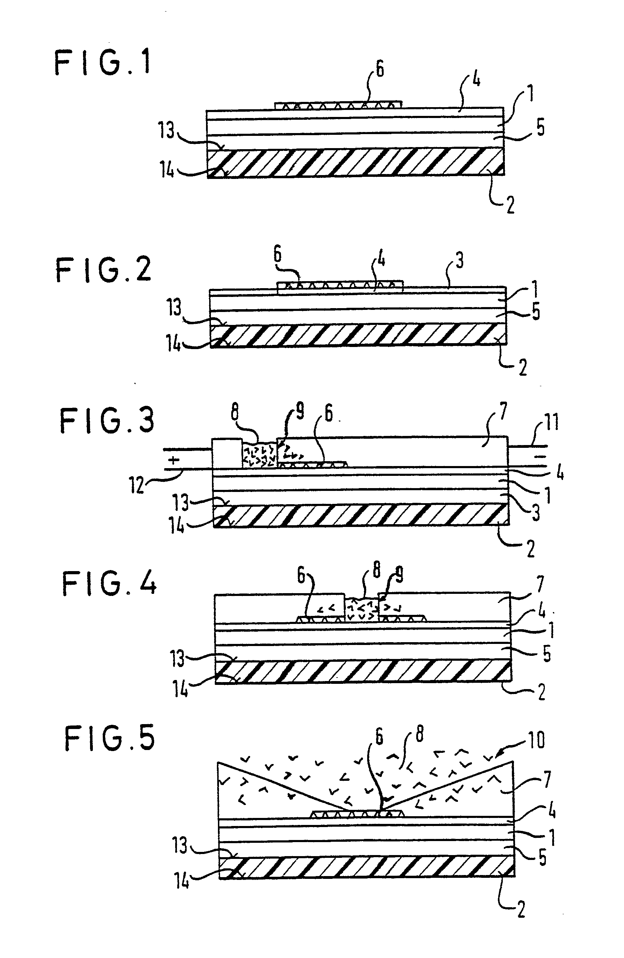

In den Figuren 1 und 2 besteht der Nachweiskörper bzw. Schichtkörper aus einem Trägerplättchen 2, einer darüber befindlichen Dielektrikumsschicht 1 aus SiO$_2$, welche mit einer Nachweisreaktionsmittelschicht 4 beschichtet ist. Diese Schicht 4 kann mit der nachzuweisenden Substanz z. B. eine biomolekulare Reaktion, insbesondere eine Enzym-Trägerreaktion, Toxin-Rezeptorreaktion, Antigen-Antikörperreaktion oder dgl. ausführen. Nach Durchführung des Nachweisverfahrens bildet sich auf der Nachweisreaktionsmittelschicht 4 eine z. B. durch Bindung entstandene Schicht 6 der nachzuweisenden Substanz z. B. aus dem entsprechenden Antikörper bzw. Antigen.

Beim Ausführungsbeispiel in der Fig. 2 ist die Nachweisreaktionsmittelschicht 4 inselförmig ausgebildet und wird von einer nichtaktiven organischen Schicht 3, welche die gleichen optischen Eigenschaften wie die Nachweisreaktionsmittelschicht 4 aufweist, umgeben. Diese Schichten 3 und 4 sind monomolekular aufgebracht.

Zwischen der dielektrischen Schicht 1 und dem Trägerkörper 2 befindet sich eine weitere dielektrische Schicht 5, die beim Ausführungsbeispiel aus SiO besteht. Es können auch mehrere derartige zusätzliche dielektrische Schichten 5 vorgesehen sein. Die Schichtdicken der einzelnen Schichten 1, 3, 4, 5 sind so bemessen, daß die Gesamtschichtanordnung dann, wenn eine zusätzliche Schicht in Form der nachzuweisenden Schicht 6 monomolekular z. B. durch Bindung angelagert wird, im Bereich der angelagerten nachzuweisenden Substanzschicht 6 einen Farbumschlag bewirkt. Dieser Farbumschlag wird in bevorzugter Weise in den für das Auge empfindlichsten Wellenlängenbereich gelegt. Gleichzeitig ist es möglich, die äußere Oberflächenschicht so auszubilden, daß sie die gewünschten chemischen und physikalischen Eigenschaften für die Nachweisreaktion besitzt.

Als Trägerkörper 2 kann z. B. Glas, Silicium oder Kunststoff verwendet werden. Das Trägerkörpermaterial ist so ausgebildet, daß es lichtabsorbierend wirkt. Die Lichtabsorption kann über den ganzen Trägerkörper hin erfolgen oder innerhalb einer bestimmten Schicht des Trägerkörpers stattfinden. Es wird hierdurch sichergestellt, daß kein Licht an der rückwärtigen Oberfläche 14 des Trägerkörpers 2 reflektiert wird, sondern nur an der mit der Schichtanordnung versehenen Oberfläche 13 des Trägerkörpers.

Bei den in den Figuren 3 bis 5 dargestellten Ausführungsbeispielen erfolgt der Transport der nachzuweisenden Substanz durch einen Gelkörper 7, welcher auf die Nachweisreaktionsmittelschicht 4 aufgebracht ist.

Beim Ausführungsbeispiel der Figur 3 ist im Gelkörper 7 eine behälterförmige Ausnehmung 9 eingeformt, die mit einer die nachzuweisende Substanz enthaltenden Probenlösung 8 angefüllt ist. Die nachzuweisende Substanz wird mit Hilfe von Elektrophorese, bei der durch Elektroden 11 und 12 im Gelkörper ein elektrisches Feld erzeugt wird, entlang der Oberfläche der Nachweisreaktionsmittelschicht 4 durch den Gelkörper 7 transportiert. Die Transportrichtung hängt von der Richtung des elektrostatischen Feldes, das an den Gelkörper 7 angelegt ist, ab.

Beim Ausführungsbeispiel der Figur 4 sind eine oder mehrere behälterartige Ausnehmungen 9 im Gelkörper 7 vorgesehen. Der Transport der nachzuweisenden Substanz erfolgt durch Diffusion in radialer Richtung, ausgehend von der in der Ausnehmung 9 vorhandenen Probenlösung 8. Der Transport erfolgt entlang der Oberfläche der Nachweisreaktionsmittelschicht 4.

Beim Ausführungsbeispiel der Figur 5 ist der Gelkörper 7 mit keilförmigem Querschnitt auf der Nachweisreaktionsmittelschicht 4 aufgebracht. Die Probenlösung 8 mit der nachzuweisenden Substanz befindet sich über dem Gelkörper 7 mit dem keilförmigen Querschnitt. Der Transport der nachzuweisenden Substanz erfolgt im Gelkörper in etwa senkrechter Richtung zur Oberfläche der Nachweisreaktionsmittelschicht 4.

Hierbei reagiert die nachzuweisende Substanz zuerst an der Stelle, an der der Gelkörper 7 am dünnsten ausgebildet ist. Im Verlauf der Zeit erfolgt auch an den Stellen, an denen der Gelkörper dicker ausgebildet ist, eine Reaktion zwischen der nachzuweisenden Substanz und der Nachweisreaktionsmittelschicht 4. Dies hat seinen Grund darin, daß die Nachweisgrenze abhängt von der Dicke des Gelkörpers 7. Es läßt sich daher, wenn die Nachweisreaktion innerhalb einer bestimmten Zeit durchgeführt wird, eine Bestimmung der Konzentration bzw. der Menge der nachzuweisenden Substanz in der Probenlösung 8 durchführen, wobei die geometrische Abmessung der Reaktionszone, innerhalb welcher die nachzuweisende Substanz als Schicht 6 auf der Nachweisreaktionsmittelschicht 4 vorhanden ist, ein Maß abgibt.

Die Menge der nachzuweisenden Substanz kann durch Standardkurven, welche beim Messen bekannter Probenlösungen gewonnen wurden, durch Ausmessen der Reaktionszonen erhalten werden. Es ist damit ohne weiteres auch ein quantitativer Nachweis möglich.

**Patentansprüche**

1. Aus mehreren Schichten bestehender Schichtkörper zum Nachweis und/oder Messen der Konzentration einer chemischen Substanz, insbesondere biologischer Herkunft in einem Medium, z. B. Wasser, Gel, Blutserum, mit einem nichtmetallischen Träger (2), der mit einer dielektrischen Schicht (1) versehen ist, auf welcher ein mit der nachzuweisenden Substanz reagierendes Nachweisreaktionsmittel aufgebracht ist, auf welchem sich beim Einbringen in das zu untersuchende Medium eine optisch nachweisbare Schicht der nachzuweisenden Substanz bildet, dadurch gekennzeichnet, daß zwischen dem Träger (2), bei dessen dielektrischem Brechungsindex der Imaginärteil gegenüber dem Realteil vernachläßigbar klein ist, und der dielektrischen Schicht (1), welche aus $SiO_2$ besteht, eine oder mehrere dielektrische, gegenüber dem Träger (2) und der dielektrischen Schicht (1) einen unterschiedlichen Brechungsindex aufweisende Schicht (5) bzw. Schichten angeordnet ist bzw. sind, durch welche die Gesamtschichtanordnung, bestehend aus den dielektrischen Schichten (1, 5), der Nachweisreaktionsmittelschicht (4) und der nachzuweisenden Substanzschicht (6) für nichtmonochromatisches, insbesondere weißes Licht, welches an der mit der Schichtanordnung versehenen Oberfläche (13) des Trägers (2) reflektiert wird, im Wellenlängenbereich von 525-600 mm reflexionsmindernd ausgebildet ist.

2. Schichtkörper nach Anspruch 1, dadurch gekennzeichnet, daß das Nachweisreaktionsmittel (4) nur stellenweise auf der dielektrischen Schicht (1) vorgesehen ist.

3. Schichtkörper nach Anspruch 1 oder 2, dadurch gekennzeichnet, daß die Nachweisreaktionsmittelschicht (4) auf der dielektrischen Schicht (1) inselförmig vorgesehen ist und die inselförmige Nachweisreaktionsmittelschicht (4) von einer nichtaktiven organischen Substanzschicht (3) gleicher Dicke und optischer Eigenschaften umgeben ist.

4. Schichtkörper nach einem der Ansprüche 1 bis 3, dadurch gekennzeichnet, daß zur Bindung bzw. Adsorption der Nachweisreaktionsmittelschicht (4) die dielektrische $SiO_2$-Schicht mit Organo-Siliciumverbindungen behandelt ist.

5. Schichtkörper nach einem der Ansprüche 1 bis 4, dadurch gekennzeichnet, daß die Nachweisreaktionsmittelschicht (4) aus einem Antigen oder Antikörper gebildet ist.

6. Schichtkörper nach einem der Ansprüche 1 bis 5, dadurch gekennzeichnet, daß die Nachweisreaktionsmittelschicht (4) monomolekular auf die dielektrische Schicht (1) aufgebracht ist.

7. Schichtkörper nach einem der Ansprüche 1 bis 6, dadurch gekennzeichnet, daß der Träger lichtabsorbierend ausgebildet ist.

8. Schichtkörper nach einem der Ansprüche 1 bis 7, dadurch gekennzeichnet, daß die dielektrische Zwischenschicht (5) aus SiO besteht.

9. Schichtkörper nach einem der Ansprüche 1 bis 8, dadurch gekennzeichnet, daß der Träger (2) einen Brechungsindex von 1,45 bis 1,90 aufweist.

10. Schichtkörper nach einem der Ansprüche 1 bis 9, dadurch gekennzeichnet, daß der Träger (2) aus dunkel eingefärbtem Glas oder dunkel eingefärbtem Kunststoff oder Silicium besteht.

11. Schichtkörper nach einem der Ansprüche 1 bis 10, dadurch gekennzeichnet, daß er plättchenförmig ausgebildet ist.

12. Verfahren zum Nachweis einer biochemisch reaktionsfähigen Substanz, insbesondere bei einem immunologischen Verfahren, bei dem die nachzuweisende Substanz durch Reaktion mit einer auf einem aus mehreren Schichten bestehenden Schichtkörper vorhandenen Nachweisreaktionsmittelschicht in einem Medium wie Wasser, Gel, Blutserum zur Reaktion gebracht wird, bei der die nachzuweisende Substanz sich als optisch nachweisbare Schicht auf dem Schichtkörper bildet, dadurch gekennzeichnet, daß die nachzuweisende Substanzschicht auf einem Schichtkörper nach einem der Ansprüche 1 bis 11 gebildet wird und dann zur Erzielung einer visuell feststellbaren Farbänderung am Schichtkörper bzw. Schichtkörperteilen mit der am Schichtkörper vorhandenen nachzuweisenden Substanzschicht gegenüber dem Schichtkörper bzw. den Schichtkörperteilen ohne die nachzuweisende Substanzschicht weißes Licht oder nichtmonochromatisches Licht an der mit der Schichtanordnung versehenen Trägeroberfläche des Schichtkörpers zur Reflektion gebracht wird.

13. Verfahren nach Anspruch 12, dadurch gekennzeichnet, daß die nachzuweisende Substanz unter Zuhilfenahme von Diffusion oder Elektrophorese durch Transport in einem auf dem Schichtkörper aufgebrachten Gelkörper längs oder quer zur Nachweisreaktionsmittelschicht mit dieser zur Reaktion gebracht wird, und der Gelkörper für den optischen Nachweis entfernt wird.

14. Verfahren nach Anspruch 12 oder 13, dadurch gekennzeichnet, daß die geometrische Ausdehnung des Flächenbereichs, in welchem die nachzuweisende Substanz als Schicht sich bildet, ein Maß für deren Konzentration bzw. Menge ist.

15. Verfahren nach einem der Ansprüche 12 bis 14, dadurch gekennzeichnet, daß die Probenlösung, welche die nachzuweisende Substanz enthält, in einem oder mehrere behälterartige Ausnehmungen des Gelkörpers eingebracht wird und durch Diffusion die Probenlösung in radialer Richtung aus der behälterartigen Ausnehmung in den Gelkörper transportiert wird.

16. Verfahren nach einem der Ansprüche 12 bis 15, dadurch gekennzeichnet, daß der Gelkörper mit unterschiedlicher Dicke auf den Schichtkörper aufgebracht wird und daß die Konzentration bzw. Menge der nachzuweisenden Substanz bestimmt wird aus der Lage bzw. den geometrischen Abmessungen des Flächenbereichs auf dem Schichtkörper, in welchem die Farbänderung nach einer bestimmten Reaktionszeit stattfindet.

17. Verfahren nach Anspruch 16, dadurch gekennzeichnet, daß der Gelkörper mit keilförmigen Querschnitt auf den Schichtkörper aufgebracht wird.


**Claims**

1. A layer member comprising a plurality of layers, for detecting and/or measuring the concentration of a chemical substance, in particular of biological origin, in a medium, for example water, gel or blood serum, having a non-metal carrier (2) which is provided with a dielectric layer (1) on which there is a detection reactant reacting with the substance to be detected, an optically detectable layer of the substance to be detected being formed on the detection reactant upon being introduced into the medium to be investigated, characterised in that arranged between the carrier (2) in whose dielectric refractive index the imaginary portion is negligibly small in comparison with the real portion, and the dielectric layer (1) which comprises $SiO_2$ is one or more dielectric layers (5) which have a different refractive index from the carrier (2) and the dielectric layer (1) and by virtue of which the entire layer arrangement consisting of the dielectric layers (1, 5), the detection reactant layer (4) and the layer (6) of substance to be detected is adapted to reduce reflection in respect of non-monochromatic and in particular white light which is reflected at the surface (13) of the carrier (2), which is provided with the layer arrangement, in the wavelength range of from 525 to 600 nm.

2. A member according to claim 1 characterised in that the detection reactant (4) is only provided on the dielectric layer (1) at various locations thereon.

3. A member according to claim 1 or claim 2 characterised in that the detection reactant layer (4) is provided on the dielectric layer (1) in an island configuration and the island-like detection reactant layer (4) is surrounded by a layer (3) of non-active organic substance of the same thickness and optical properties.

4. A member according to one of claims 1 to 3 characterised in that the dielectric $SiO_2$ layer is treated with organo-silicon compounds for the purposes of binding or adsorption of the detection reactant layer (4).

5. A member according to one of claims 1 to 4 characterised in that the detection reactant layer (4) is formed from an antigen or antibody.

6. A member according to one of claims 1 to 5 characterised in that the detection reactant layer (4) is applied to the dielectric layer (1) in monomolecular form.

7. A member according to one of claims 1 to 6 characterised in that the carrier is light-absorbing.

8. A member according to one of claims 1 to 7 characterised in that the dielectric intermediate layer (5) comprises $SiO_2$.

9. A member according to one of claims 1 to 8 characterised in that the carrier (2) has a refractive index of from 1.45 to 1.90.

10. A member according to one of claims 1 to 9 characterised in that the carrier (2) comprises dark-coloured glass or dark-coloured plastics material or silicon.

11. A member according to one of claims 1 to 10 characterised in that it is of plate-like configuration.

12. A method of detecting a biochemically reactive substance, in particular in an immunological process, in which the substance to be detected is caused to react by reaction with a layer of detection reactant present on a layer member comprising a plurality of layers, in a medium such as water, gel or blood serum, wherein the substance to be detected is formed as an optically detectable layer on the layer member, characterised in that the layer of susbtance to be detected is formed on a layer member according to one of claims 1 to 11 and then, to produce a visually detectable change in colour on the layer member or layer member portions with the layer of substance to be detected, which is present on the layer member, relative to the layer member or the layer member portions without the layer of substance to be detected, white light or non-monochromatic light is caused to be reflected at the carrier surface of the layer member, which is provided with the layer arrangement.

13. A method according to claim 12 characterised in that the substance to be detected is caused to react with the layer of detection reactant, by means of diffusion or electrophoresis by transportation in a gel body applied to the layer member, along or transversely relative to the layer of detection reactant, and the gel body is removed for the optical detection step.

14. A method according to claim 12 or claim 13 characterised in that the geometrical extent of the surface region in which the substance to be detected is formed as a layer is a measurement in respect of the concentration or amount thereof.

15. A method according to one of claims 12 to 14 characterised in that the test solution which contains the substance to be detected is introduced into one or more container-like openings in the gel body and by diffusion the test solution is transported in a radial direction out of the container-like opening into the gel body.

16. A method according to one of claims 12 to 15 characterised in that the gel body is applied to the layer member in different thicknesses and that the concentration or amount of the substance to be detected is determined from the position or the geometrical dimensions of the surface region on the layer member in which the change in colour takes place after a given reaction time.

17. A method according to claim 16 characterised in that the gel body is applied to the layer member in a wedge-shaped cross-section.

### Revendications

1. Corps stratifié constitué de plusieurs couches pour détecter et/ou mesurer la concentration d'une substance chimique, notamment d'origine biologique, dans un milieu, par exemple de l'eau, un gel, du sérum sanguin, avec un support non métallique (2) muni d'une couche (1) diélectrique sur laquelle est appliqué un réactif de détection réagissant avec la substance à détecter, sur lequel se forme, lorsqu'on l'introduit dans le milieu à examiner, une couche de la substance à détecter optiquement détectable, caractérisé en ce qu'on dispose entre le support (2), pour l'indice de réfraction diélectrique duquel la partie imaginaire est négligeable par rapport à la partie réelle, et la couche diélectrique (1) constituée de $SiO_2$, une ou plusieurs couches diélectriques ayant un indice de réfraction différent de celui du support (2) et de celui de la couche diélectrique (1) qui permettent de réduire la réflexion de l'ensemble multicouches, constitué par les couches diélectriques (1, 5), la couche de réactif de détection (4) et la couche de la substance à détecter (6), pour la lumière non monochromatique, notamment la lumière blanche qui est réfléchie sur la surface (13) du support (2), notamment dans la plage des longueurs d'onde entre 525 et 600 mm.

2. Corps stratifié selon la revendication 1, caractérisé en ce qu'on prévoit le réactif de détection (4) seulement par places sur la couche diélectrique (1).

3. Corps stratifié selon la revendication 1 ou 2, caractérisé en ce que la couche de réactif de détection (4) est prévue par îlots sur la couche diélectrique (1) et les îlots de la couche de réactif de détection (4) sont entourés par une couche de substance organique inerte (3) ayant la même épaisseur et les mêmes propriétés optiques.

4. Corps stratifié selon l'une des revendications 1 à 3, caractérisé en ce que, pour la liaison ou l'adsorption de la couche de réactif de détection (4), la couche diélectrique de $SiO_2$ est traitée avec des composés organosiliciques.

5. Corps stratifié selon l'une des revendications 1 à 4, caractérisé en ce que la couche de réactif de détection (4) est formée par un antigène ou un anticorps.

6. Corps stratifié selon l'une des revendications 1 à 5, caractérisé en ce que la couche de réactif de détection (4) est appliquée monomoléculairement sur la couche diélectrique (1).

7. Corps stratifié selon l'une des revendications 1 à 6, caractérisé en ce que le support est réalisé absorbant la lumière.

8. Corps stratifié selon l'une des revendications 1 à 7, caractérisé en ce que la couche intermédiaire diélectrique (5) est en $SiO_2$.

9. Corps stratifié selon l'une des revendications 1 à 8, caractérisé en ce que le support (2) a un indice de réfraction compris entre 1,45 et 1,90.

10. Corps stratifié selon l'une des revendications 1 à 9, caractérisé en ce que le support (2) est en verre de couleur foncée, en matière plastique de couleur foncée ou en silicium.

11. Corps stratifié selon l'une des revendications 1 à 10, caractérisé en ce qu'il est réalisé sous forme de plaquette.

12. Procédé pour détecter une substance biochimiquement réactive, notamment dans un procédé immunologique, dans lequel la substance à détecter est amenée à réagir dans un milieu tel que l'eau, un gel, du sérum sanguin, avec une couche de réactif de détection se trouvant sur un corps stratifié constitué de plusieurs couches, réaction au cours de laquelle la substance à détecter forme sur le corps stratifié une couche optiquement détectable, caractérisé en ce que la couche de substance à détecter est formée sur un corps stratifié selon l'une des revendications 1 à 11, puis, pour obtenir un changement de couleur détectable à l'œil du corps stratifié (ou de parties de corps stratifié) revêtu de la couche de substance à détecter par rapport au corps stratifié (ou aux parties de corps) dépourvu de la couche de substance à détecter, on fait réfléchir de la lumière blanche ou de la lumière non monochromatique sur la surface support du corps stratifié revêtu de cette couche.

13. Procédé selon la revendication 12, caractérisé en ce que la substance à détecter est amenée à réagir avec la couche de réactif de détection en étant transportée le long de cette couche ou transversalement à elle dans un corps de gel appliqué sur le corps stratifié, par diffusion ou électrophorèse, et le corps de gel est retiré pour permettre la détection optique.

14. Procédé selon la revendication 12 ou 13, caractérisé en ce que l'étendue géométrique de la zone de surface dans laquelle la substance à détecter se forme en couche, est une mesure pour la concentration ou la quantité de celle-ci.

15. Procédé selon l'une des revendications 12 à 14, caractérisé en ce que la solution échantillon contenant la substance à détecter est introduite dans une ou plusieurs cavités en forme de récipients du corps de gel et la solution échantillon est transportée dans le corps de gel par diffusion dans le sens radial à partir de la cavité en forme de récipient.

16. Procédé selon l'une des revendications 12 à 15, caractérisé en ce que le corps de gel est appliqué sur le corps stratifié avec un épaisseur variable, et la concentration ou la quantité de la substance à détecter est déterminée à partir de la position ou des dimensions géométriques de la zone de surface sur le corps stratifié dans laquelle se produit le changement de couleur après un temps de réaction déterminé.

17. Procédé selon la revendication 16, caractérisé en ce que le corps de gel est appliqué sur le corps stratifié avec une section transversale cunéiforme.

FIG.1

FIG.2

FIG.3

FIG.4

FIG.5